(19) **Europäisches Patentamt European Patent Office Office européen des brevets**

(11) **EP 4 751 653 A1**

(12) **EUROPEAN PATENT APPLICATION**

(43) Date of publication:
03.06.2026 Bulletin 2026/23

(21) Application number: 25219062.4

(22) Date of filing: 27.11.2025

(51) International Patent Classification (IPC):
**A61B 6/04** (2006.01) **A61B 6/00** (2024.01)

(52) Cooperative Patent Classification (CPC):
**A61B 6/0407; A61B 6/54;** A61B 6/03; A61B 6/037;
A61B 6/102; A61B 6/4258; A61B 6/4266;
A61B 6/467; A61B 6/488; A61B 6/505;
A61B 6/5241; A61B 6/5264; A61B 6/541;
A61B 6/547; A61B 6/56; (Cont.)

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH LA MA MD TN**

(30) Priority: 27.11.2024 CN 202411718745

(71) Applicant: **Shanghai United Imaging Healthcare Co., Ltd.**
**Shanghai 201807 (CN)**

(72) Inventors:
• **SHI, Lei**
  **Shanghai, 201807 (CN)**
• **LI, Dianze**
  **Shanghai, 201807 (CN)**
• **LI, Zeshu**
  **Shanghai, 201807 (CN)**
• **ZHAO, Yumo**
  **Shanghai, 201807 (CN)**
• **CHEN, Yang**
  **Shanghai, 201807 (CN)**

(74) Representative: **Wang, Bo**
**Panovision IP**
**Ebersberger Straße 3**
**85570 Markt Schwaben (DE)**

(54) **SYSTEMS AND METHODS FOR SCANNING SUBJECTS**

(57) The present disclosure provides a system and method for scanning a subject. The method includes determining reference dimensions, along the width direction of the table, of the subject on the table at positions along the moving direction of the table; dividing, based on the reference dimensions, the subject into at least one region along the moving direction of the table, the at least one region including at least one of a first region or a second region; and controlling a relative motion between the detector and the table to perform scanning on the at least one region in sequence along the moving direction of the table. During scanning one of the at least one region, the detector moves along the width direction of the table relative to the table.

_300_

Determining dimensions, along the width direction of the table, of the subject on the table at positions along the moving direction of the table — 310

Dividing, based on the dimensions, the subject into at least one region along the moving direction of the table — 320

Controlling a relative motion between the probe and the table to perform a scanning on the at least one region in sequence along the moving direction of the table — 330

**FIG. 3**

**EP 4 751 653 A1**

(52) Cooperative Patent Classification (CPC): (Cont.)
G01N 23/046

**Description**

**CROSS-REFERENCE TO RELATED APPLICATIONS**

This application claims the priority of Chinese Patent Application No. 202411718745.1, filed on November 27, 2024, the contents of which are hereby incorporated by reference.

**TECHNICAL FIELD**

The disclosure generally relates to the technology field of medical device, and more particularly relates to systems and methods for scanning subjects.

**BACKGROUND**

Scanning devices are used to scan patients to obtain anatomical or physiological information of the patients. For example, for a dual-detector single-photon emission computed tomography (SPECT) device, the primary purpose of scans conducted with the dual-detector SPECT device is to facilitate one-time scanning for acquiring anterior and posterior SPECT projection images of the patient, allowing for the assessment of whole-body metabolic situations-commonly seen in whole-body bone scans.

A detector of a scanning device is usually fixed to a gantry, and a scanning field of view along a width of the patient's body is limited. The scanning width of the detector is related to its physical width. This means that for patients requiring an ultra-wide field of view (such as obese individuals), additional scans beyond the standard field of view must be performed. For example, patients may need to turn to left and right sides separately for rescanning. However, left and right lateral scans compromise patient comfort and accuracy during scanning, while also resulting in lower scanning efficiency.

Thus, it is desirable to provide systems and methods for scanning a subject with improved scanning efficiency and accuracy.

**SUMMARY**

According to a first aspect of the present disclosure, a method for scanning a subject is provided. The method is implemented on a scanning system having a table capable of moving and a detector capable of moving along at least one of a moving direction of the table or a width direction of the table. The method includes determining reference dimensions, along the width direction of the table, of the subject on the table at positions along the moving direction of the table; dividing, based on the reference dimensions, the subject into at least one region along the moving direction of the table; and controlling a relative motion between the detector and the table to perform scanning on the at least one region in sequence along the moving direction of the table. The at least one region includes at least one of a first region or a second region. The first region includes a region where a dimension of the subject along the width direction of the table is smaller than or equal to a preset scanning width of the detector along the width direction of the table, and the second region includes a region where a dimension of the subject along the width direction of the table is greater than the preset scanning width of the detector along the width direction of the table.

According to a second aspect of the present disclosure, a single-photon emission computed tomography (SPECT) system is provided. The SPECT device includes: a gantry; a table configured to carry a subject and move relative to the gantry; a detector disposed on the gantry and including at least two grammar detectors; and a processor. The processor is configured to: acquire a contour of the subject carried on the table, determine a truncated field of view (FOV) of the detector based on the contour, and control the at least two grammar detectors to move to positions corresponding to multiple viewing angles relative to the table in the truncated FOV, so as to restore the truncated FOV to a full FOV.

According to a third aspect of the present disclosure, a single-photon emission computed tomography (SPECT) system is provided. The SPECT device includes: a gantry; a table configured to carry a subject and move relative to the gantry; a detector disposed on the gantry and including a first gamma detector and a second gamma detector arranged opposite to each other; and a processor. The processor is configured to: obtain a contour of the subject lied on the table, determine a truncated field of view (FOV) of the detector based on the contour, and control a relative motion between the detector and the table to restore the truncated FOV to a full FOV.

**BRIEF DESCRIPTION OF THE DRAWINGS**

The present disclosure is further described in terms of exemplary embodiments. These exemplary embodiments are described in detail with reference to the drawings. The drawings are not scaled. These embodiments are non-limiting exemplary embodiments, in which like reference numerals represent similar structures throughout the several views of the drawings, and wherein:

FIG. 1A is a schematic diagram illustrating an exemplary scanning system according to some embodiments of the present disclosure;
FIG. 1B shows a truncated image of a subject according to some embodiments of the present disclosure;
FIG. 2 is a block diagram illustrating an exemplary processing device according to some embodiments of the present disclosure;
FIG. 3 is a schematic flowchart illustrating an exemplary process for scanning a subject according to some embodiments of the present disclosure;

FIG. 4A shows an exemplary contour of a subject on a table according to some embodiments of the present disclosure;

FIG. 4B is a schematic diagram illustrating an exemplary moving process of a detector according to some embodiments of the present disclosure;

FIG. 4C is a schematic diagram illustrating an exemplary moving process of a detector according to some embodiments of the present disclosure;

FIG. 4D shows exemplary sensors on a detector according to some embodiments of the present disclosure;

FIG. 4E is a schematic diagram illustrating collision between a subject and an inner wall of a bore of an SPECT device according to some embodiments of the present disclosure;

FIG. 5 is a schematic diagram illustrating a process for scanning a subject having a plurality of regions along a moving direction of a table according to some embodiments of the present disclosure;

FIG. 6 is a block diagram illustrating an exemplary processing device according to some embodiments of the present disclosure; and

FIG. 7 is a schematic flowchart illustrating an exemplary process for scanning a subject according to some embodiments of the present disclosure.

**DETAILED DESCRIPTION**

The following description is presented to enable any person skilled in the art to make and use the present disclosure and is provided in the context of a particular application and its requirements. Various modifications to the disclosed embodiments will be readily apparent to those skilled in the art, and the general principles defined herein may be applied to other embodiments and applications without departing from the spirit and scope of the present disclosure. Thus, the present disclosure is not limited to the embodiments shown but is to be accorded the widest scope consistent with the claims.

The terminology used herein is for the purpose of describing particular example embodiments only and is not intended to be limiting. As used herein, the singular forms "a," "an," and "the" may be intended to include the plural forms as well, unless the context clearly indicates otherwise. It will be further understood that the terms "comprise," "comprises," and/or "comprising," "include," "includes," and/or "including" when used in this disclosure, specify the presence of stated features, integers, steps, operations, elements, and/or components, but do not preclude the presence or addition of one or more other features, integers, steps, operations, elements, components, and/or groups thereof.

It will be understood that, although the terms "first," "second," "third," etc., may be used herein to describe various elements, these elements should not be limited by these terms. These terms are only used to distinguish one element from another. For example, a first element could be termed a second element, and, similarly, a second element could be termed a first element, without departing from the scope of exemplary embodiments of the present disclosure.

These and other features, and characteristics of the present disclosure, as well as the methods of operation and functions of the related elements of structure and the combination of parts and economies of manufacture, may become more apparent upon consideration of the following description with reference to the accompanying drawings, all of which form a part of this disclosure. It is to be expressly understood, however, that the drawings are for the purpose of illustration and description only and are not intended to limit the scope of the present disclosure. It is understood that the drawings are not to scale.

In the present disclosure, it should be understood that terms such as "center," "longitudinal," "transverse," "length," "width," "thickness," "upper," "lower," "front," "rear," "left," "right," "vertical," "horizontal," "top," "bottom," "inner," "outer," "clockwise," "counterclockwise," "axial," "radial," "circumferential," etc., are used to indicate orientations or positional relationships based on those shown in the accompanying drawings. These terms are used solely to facilitate the description of this application and to simplify the description, and do not indicate or imply that the referred devices or elements must have a specific orientation or be constructed and operated in a specific orientation. Therefore, they should not be construed as limitations on this application.

The flowcharts used in the present disclosure illustrate operations that systems implement according to some embodiments in the present disclosure. It is to be expressly understood, the operations of the flowchart may be implemented not in order. Conversely, the operations may be implemented in an inverted order, or simultaneously. Moreover, one or more other operations may be added to the flowcharts. One or more operations may be removed from the flowcharts.

The term "image" in the present disclosure is used to collectively refer to imaging data (e.g., scan data, projection data) and/or images of various forms, including a two-dimensional (2D) image, a three-dimensional (3D) image, a four-dimensional (4D), etc. The term "pixel" and "voxel" in the present disclosure are used interchangeably to refer to an element of an image. The term "region," "location," and "area" in the present disclosure may refer to a location of an anatomical structure shown in the image or an actual location of the anatomical structure existing in or on a target object's body, since the image may indicate the actual location of a certain anatomical structure existing in or on the target object's body.

Provided herein are a system and method for scanning a subject. The method may be implemented on a scanning system having a table capable of moving and a detector capable of moving along at least one of a moving direction of the table or a width direction of the table. The method may include determining reference dimensions, along the width direction of the table, of the subject on the table at positions along the moving direction of the table. The method may further include dividing, based on the reference dimensions, the subject into at least one region along the moving direction of the table. The at least one region may include at least one of a first region or a second region. The first region may include a region where a dimension of the subject along the width direction of the table is smaller than or equal to a preset scanning width of the detector along the width direction of the table, and the second region may include a region where a dimension of the subject along the width direction of the table is greater than the preset scanning width of the detector along the width direction of the table. The method may further include controlling a relative motion between the detector and the table to perform scanning on the at least one region.

According to some embodiments of the present disclosure, since a scanning range (or scanning field of view) of the detector along the width direction of the table is generally limited, when scanning a subject (such as an obese individual) having an ultra-wide body, by dividing the subject into at least one region along the moving direction of the table based on the reference dimensions and controlling the relative motion between the detector and the table to perform the scanning on the at least one region, some regions having dimensions along the width direction of the table greater than the preset scanning width of the detector can also be scanned. Thus ,the subject's whole-body images can be acquired in a single scan without the need for hardware modifications (such as increasing detector width), for extended acquisition time, and/or for subject repositioning. This eliminates the need for additional scans beyond the standard field of view or repositioning the subject, thereby enhancing subject's comfort during examinations, reducing acquisition time, and increasing hospital's examination throughput.

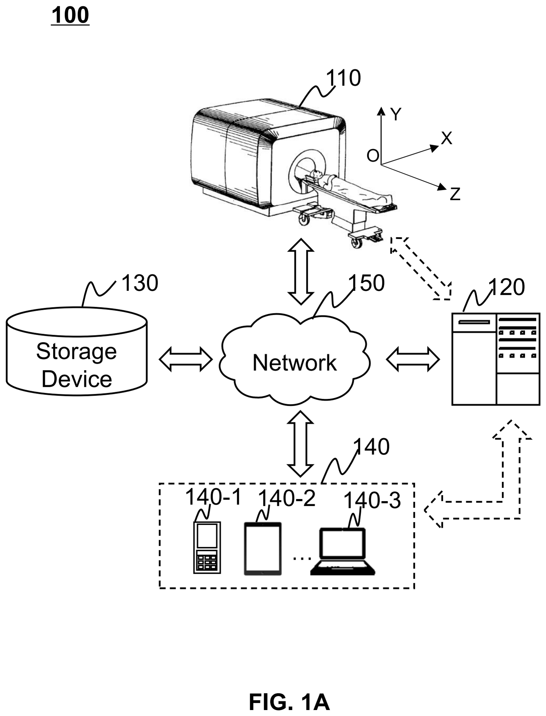

FIG. 1A is a schematic diagram illustrating an exemplary scanning system 100 according to some embodiments of the present disclosure. As illustrated in FIG. 1A, the scanning system 100 may include a medical device 110, a processing device 120, a storage device 130, a terminal device 140, and a network 150. The components in the scanning system 100 may be connected to and/or communicate with each other via a wireless connection, a wired connection, or a combination thereof.

The medical device 110 may be configured to acquire imaging data relating to at least one part of a subject. The imaging data may include an image (e.g., an image slice), projection data, or a combination thereof. The subject may be biological or non-biological. For example, the subject may include a patient, a man-made object, etc. As another example, the subject may include a specific portion, organ, and/or tissue of the patient. For example, the subject may include the head, the neck, the thorax, the heart, the stomach, a blood vessel, soft tissue, a tumor, nodules, or the like, or any combination thereof. In some embodiments, the medical device 110 may include a single-photon emission computed tomography (SPECT) device, an SPECT-magnetic resonance (SPECT-MRI) device, an SPECT-computed tomography (SPECT-CT) device, etc. For illustration purposes, an SPECT device may be taken as an example of the medical device 110.

The medical device 110 may include a gantry, one or more detectors, a detecting region, a table, or any other components. The gantry may be configured to provide support for other components (e.g., the detector(s), etc.) of the medical device 110. In some embodiments, the gantry may rotate and/or move. The detector(s) may rotate or move along with the rotation or movement of the gantry. The table may be configured to position and/or support the subject. The subject may be placed on the table and moved into or out of the detecting region of the medical device 110 along a first direction (i.e., a direction along an Z-axis or an OZ direction shown in FIG. 1A, also referred to as a length direction of the table). In some embodiments, the detector(s) may be movably arranged on the gantry along a second direction (i.e., a direction along an X-axis or an OX direction shown in FIG. 1A, also referred to as a width direction of the table). For example, the gantry is equipped with a slide rail extending along the second direction (i.e., the OX direction). Each detector is mounted on the slide rail via a sliding block, which, when moving, drives the detector to travel along the second direction (i.e., the OX direction), enabling a relative motion between the detector and the table to perform scanning on the subject. In some embodiments, the detector(s) may move along a third direction (i.e., a direction along a Y-axis or an OY direction shown in FIG. 1A, also referred to as a thickness direction of the table). For example, in order to obtain a high-resolution image of the subject, the detector(s) should be as close to the subject as possible for scanning.

In some embodiments, the detector(s) may include a plurality of gamma detectors arranged along the OX direction, each of which corresponds to a viewing angle (i.e., an angle reflecting a scanning range of the gamma detector at a postion). A position where each gamma detector is located may correspond to a viewing angle. That is, different gamma detectors may scan different ranges of the subject lied on the table. In some embodiments, scanning ranges of adjacent gamma detectors arranged along the OX direction may overlap or not overlap. In some embodiments, for scanning a certain region, when viewing angles of the gamma detectors arranged along the OX direction can form a full field of view (FOV) along the OX direction, that is, using these gamma detectors for synchronous scanning, an effective scanning width of the detector(s) along the OX direction may be greater than or equal to the maximum dimension of the certain region along the OX direction. In such cases, during the scanning, the gamma detectors and the table may be relatively fixed along the OX direction. The certain region of the subject corresponding to the full FOV may be referred to as a full FOV region. For example, the full FOV region may serve as the first region in the following text.

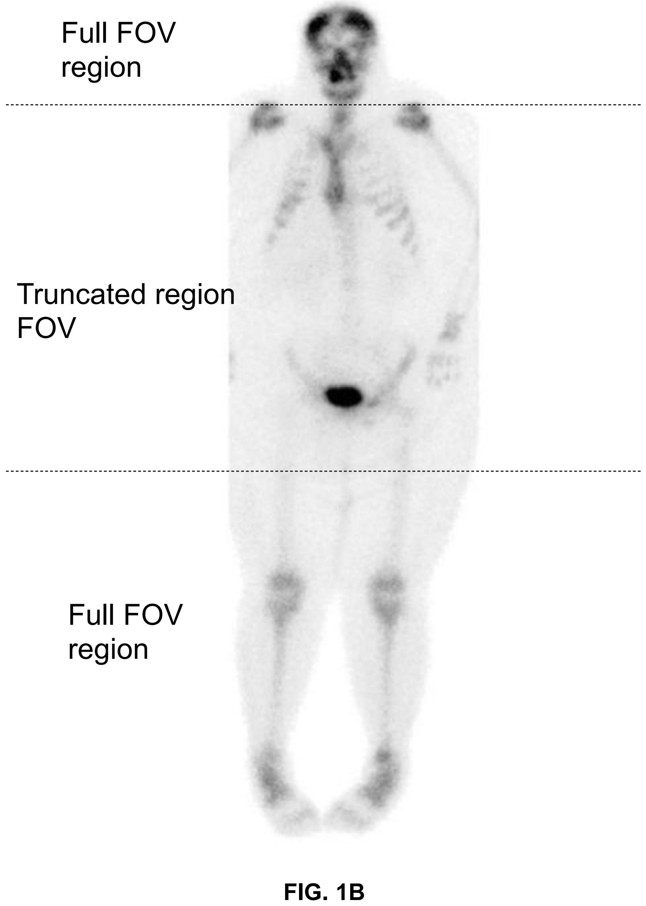

In some embodiments, for scanning a certain region, when viewing angles of the gamma detectors arranged along the OX direction can not form a full field of view (FOV) along the OX direction, that is, a truncated FOV may be formed due to the effective scanning width of the detector(s) along the OX direction is samller than the maximum dimension of the certain region along the OX direction. In such cases, during the scanning, these gamma detectors and the table may move relative to each other along the OX direction, so as to restore the truncated FOV to the full FOV. For example, the plurality of grammar detectors may be controlled to move to positions corresponding to multiple viewing angles relative to the table in the truncated FOV, so as to restore the truncated FOV to a full FOV. It should be noted that the detector(s) having the truncated FOV indicates that edge parts of the subject exceed a scanning boundary, resulting in signals not being captured by the detector(s), and ultimately presenting "edge loss" and "incomplete data" during data acquisition or image reconstruction. The certain region of the subject corresponding to the truncated FOV may be referred to as a truncated FOV region. For example, the truncated FOV region may serve as the second region in the following text. For example, FIG. 1B shows a truncated image of a subject according to some embodiments of the present disclosure. As shown in FIG. 1B, due to the head of the subject has a narrow size, a region from the head to the shoulders of the subject is a full FOV region (i.e., a first region). Due to the torso of the subject has a wide size, a region from the shoulders to the hip of the subject is a truncated FOV region (i.e., a second region). Due to the legs of the subject have a narrow size, a region from the hip to the feet of the subject is also a full FOV region (i.e., a first region). It should be noted that due to the irregular size of the human body along its axial direction, after the truncated FOVs corresponding to different regions of the subject restored to the corresponding full FOVs, viewing angles corresponding to the full FOV regions may be different.

The processing device 120 may process data and/or information relating to subject scanning to perform one or more functions described in the present disclosure. For example, the processing device 120 may determine reference dimensions, along the width direction (i.e., the OX direction) of the table, of the subject on the table at positions along the moving direction (i.e., the OZ direction) of the table. The processing device 120 may divide, based on the reference dimensions, the subject into at least one region along the moving direction of the table. Further, the processing device 120 may control a relative motion between the detector and the table to perform scanning on the at least one region. In some embodiments, the processing device 120 may be a computer, a user console, a single server or a server group, etc. The server group may be centralized or distributed. In some embodiments, the processing device 120 may be local or remote. In some embodiments, the processing device 120 may be implemented on a cloud platform. Merely by way of example, the cloud platform may include a private cloud, a public cloud, a hybrid cloud, a community cloud, a distributed cloud, an inter-cloud, a multi-cloud, or the like, or any combination thereof. In some embodiments, the processing device 120 may be implemented on a computing device or the medical device 110.

The storage device 130 may be configured to store data and/or instructions. The data and/or instructions may be obtained from, for example, the processing device 120, the medical device 110, and/or any other component of the scanning system 100. In some embodiments, the storage device 130 may store data and/or instructions that the processing device 120 may execute or use to perform exemplary methods described in the present disclosure. In some embodiments, the storage device 130 may include a mass storage device, a removable storage device, a volatile read-and-write memory, a read-only memory (ROM), or the like, or any combination thereof. In some embodiments, the storage device 130 may be implemented on the cloud platform described elsewhere in the present disclosure.

The terminal device 140 may be configured to receive information and/or data from the processing device 120, the medical device 110, and/or the storage device 130 via the network 150. For example, the terminal device 140 may receive an image from the processing device 120. In some embodiments, the terminal device 140 may provide a user interface via which a user may view information and/or input data and/or instructions to the scanning system 100. For example, the user may view, via the user interface, information associated with the medical device 110. As another example, the user may input, via the user interface, a user input instruction to set reference dimensions of the subject at positions along the moving direction of the table. In some embodiments, the terminal device 140 may include a mobile device 140-1, a tablet computer 140-2, a laptop computer 140-3, or the like, or any combination thereof. In some embodiments, the terminal device 140 may include a display that can display information in a human-readable form, such as text, image, audio, video, graph, animation, or the like, or any combination thereof.

The network 150 may facilitate the exchange of information and/or data for the scanning system 100. In some embodiments, one or more components (e.g., the medical device 110, the processing device 120, the terminal device 140, or the storage device 130) of the scanning system 100 may transmit information and/or data to one or more other components of the scanning system 100 via the network 150. In some embodiments, the network 150 may be any type of wired or wireless network, or combination thereof.

It should be noted that the above description is intended to be illustrative, and not to limit the scope of the present disclosure. Many alternatives, modifications, and variations will be apparent to those skilled in the art. The features, structures, methods, and characteristics of the exemplary embodiments described herein may be combined in various ways to obtain additional and/or alternative exemplary embodiments. In some embodiments, the scanning system 100 may include one or more additional components and/or one or more components described above may be omitted. Additionally or alternatively, two or more components of the scanning system 100 may be integrated into a single component. For example, the processing device 120 may be integrated into the medical device 110. As another example, a component of the scanning system 100 may be replaced by another component that can implement the functions of the component. However, those variations and modifications do not depart from the scope of the present disclosure.

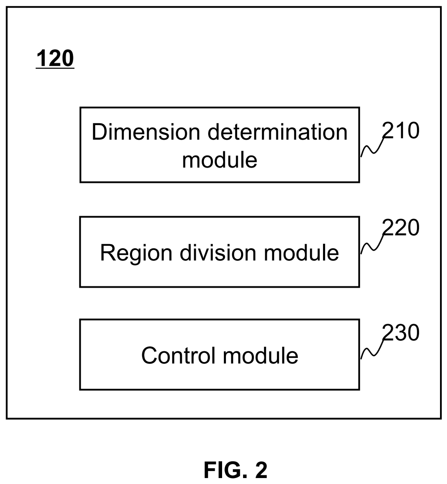

FIG. 2 is a block diagram illustrating an exemplary processing device according to some embodiments of the present disclosure. As illustrated in FIG. 2, the processing device 120 may include a dimension determination module 210, a region division module 220, and a control module 230. Each of the modules described above may be a hardware circuit that is designed to perform certain actions, e.g., according to a set of instructions stored in one or more storage media, and/or any combination of the hardware circuit and the one or more storage media.

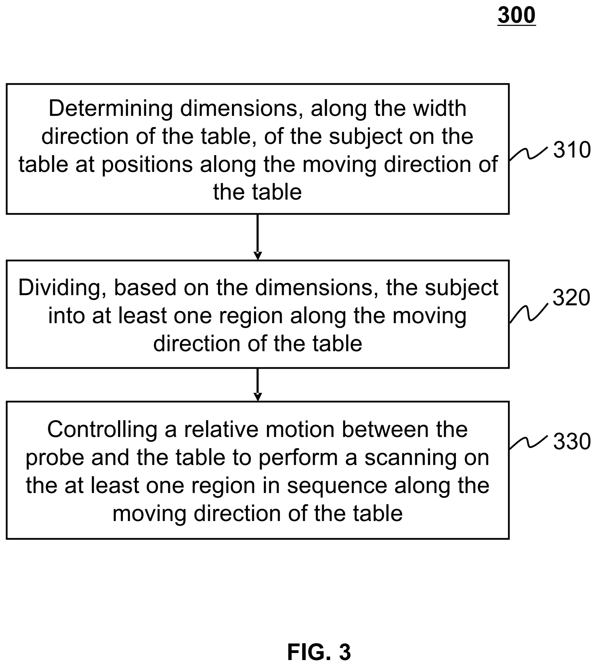

The dimension determination module 210 may be configured to determine reference dimensions, along the width direction of the table, of the subject on the table at positions along the moving direction of the table. More descriptions regarding the obtaining of the reference dimensions may be found elsewhere in the present disclosure, e.g., operation 310 in FIG. 3 and relevant descriptions thereof.

The region division module 220 may be configured to divide, based on the reference dimensions, the subject into at least one region along the moving direction of the table. In some embodiments, the at least one region may include at least one of a first region or a second region. The first region may include a region where a dimension of the subject along the width direction of the table is smaller than or equal to a preset scanning width of the detector along the width direction of the table. The second region may include a region where a dimension of the subject along the width direction of the table is greater than the preset scanning width of the detector along the width direction of the table. More descriptions regarding the dividing the subject based on the reference dimensions may be found elsewhere in the present disclosure, e.g., operation 320 in FIG. 3 and relevant descriptions thereof.

The control module 230 may be configured to control a relative motion between the detector and the table to perform scanning on the at least one region in sequence along the moving direction of the table. For example, the control module 230 may control the detector the move along a width direction of the table to scan the second region while controlling the table to remain stationary. More descriptions regarding the scanning the subject may be found elsewhere in the present disclosure, e.g., operation 330 in FIG. 3 and relevant descriptions thereof.

It should be noted that the above description is merely provided for the purposes of illustration, and is not intended to limit the scope of the present disclosure. Apparently, for persons having ordinary skills in the art, multiple variations and modifications may be conducted under the teachings of the present disclosure. However, those variations and modifications do not depart from the scope of the present disclosure. For example, the dimension determination module 210 and the region division module 220 may be integrated into a single module. As another example, some other components/modules (e.g., a storage module (not shown) for storing data) may be added into the processing device 120.

FIG. 3 is a schematic flowchart illustrating an exemplary process 300 for scanning a subject according to some embodiments of the present disclosure. In some embodiments, the process 300 may be executed by the scanning system 100. For example, the process 300 may be implemented as a set of instructions (e.g., an application) stored in the storage device 130, and the processing device 120 (e.g., the modules in FIG. 2) may execute the set of instructions and may accordingly be directed to perform the process 300. The operations of the illustrated process presented below are intended to be illustrative. In some embodiments, the process 300 may be accomplished with one or more additional operations not described and/or without one or more of the operations discussed. Additionally, the order of the operations of the process 300 illustrated in FIG. 3 and described below is not intended to be limiting.

For illustration purposes, the process 300 being implemented on an SPECT device may be described as an example. It should be noted that in some embodiments, the process 300 may also be implemented on other devices, such as an SPECT-MRI device, an SPECT-CT device, etc.

The SPECT device may have a table capable of moving along a length direction of the table and a detector capable of moving along at least one of a moving direction (also referred to as the length direction) of the table or a width direction of the table. In some embodiments, the SPECT device may be used for whole-body bone imaging, thyroid imaging, renal dynamic imaging, lymphatic imaging, myocardial imaging, etc. In some embodiments, the SPECT device may include a single-detector SPECT device, a dual-detector SPECT device, a three-detector SPECT device, etc. For example, the single-detector SPECT device may be equipped with only one detector on the gantry, which scans a subject (e.g., a patient) on the table of the single-detector SPECT device. In contrast, the dual-detector SPECT device may have detectors positioned on the gantry opposite to each other, allowing for the acquisition of anterior and posterior SPECT projection images of the subject. During scanning, either the entire subject or a portion of the subject may be scanned.

In some embodiments, the detector may include a first gamma detector and a second gamma detector. The first gamma detector and the second gamma detector may be disposed on the gantry along the width direction of the table. The first gamma detector may correspond to a first viewing angle (e.g., a viewing angle where the lefthand can be scanned), and the second gamma detector may correspond to a second viewing angle (e.g., a viewing angle where the right-hand can be scanned). In order to provide a more detailed description of the scanning manner for the subject, the scenario where the first viewing angle and the second viewing angle fail to form a full field of view (FOV) along the width direction of the table may be taken as an example. In such cases, for scanning a specific region (e.g., the following described second region), by controlling the first gamma detector to continuously move from a first position corresponding to the first viewing angle to a second position corresponding to the second viewing angle (or controlling the second gamma detector to continuously move from the second position corresponding to the second viewing angle to the first position corresponding to the first viewing angle), a full FOV may be formed. Thus, the processing device 120 may construct a complete image of the specific region of the subject only based on imaging data acquired by the first gamma detector or imaging data acquired by the second gamma detector.

It should be noted that in some embodiments, for scanning the specific region (e.g., the following described second region), the first gamma detector and the second gamma detector may be controlled to simultaneously move towards each other to form the full FOV. The first gamma detector and the second gamma detector may continuously collect gamma ray signals during their movement. Thus, the processing device 120 may construct a complete image of the specific region of the subject based on imaging data acquired by the first gamma detector and the second gamma detector. For illustration purposes, controlling one detector (e.g., the first gamma detector or the second gamma detector) to achieve the full FOV of a region of the subject may be described as an example.

In 310, the processing device 120 (e.g., the dimension determination module 210) may determine reference dimensions, along the width direction of the table, of the subject on the table at positions along the moving direction of the table.

The reference dimensions refers to specific dimensions, along the width direction of the table, of the subject that naturally lies on the table. For example, the reference dimensions include a dimension of the subject along the width direction of the table at the position corresponding to a part of the subject (e.g., the hand).

The length direction refers to a dimension based on the table, along the table's moving direction (corresponding to the OZ direction, usually the human body's head-to-feet axis), which is used to achieve the subject's scanning feeding and full-body coverage. The width direction refers to a dimension perpendicular to the length direction, along the table's transverse direction (corresponding to the OX direction, usually the human body's left shoulder-to-right shoulder/left hand-to-right hand axis), which is used for the detector to move so as to cover ultra-wide areas.

In some embodiments, the processing device 120 may obtain one or more key positions on the subject and determine the reference dimensions of the subject along the width direction of the table based on the one or more key positions. The key positions refers to positions located at the rightmost or leftmost of the subject when the subject lies on the table. For example, a patient usually has a largest width at a position corresponding to the patient's hand when the patient naturally lies on the table, thus the hand can be designated a key position. A dimension obtained by measuring the subject along the width direction of the table at the position corresponding to the hand may be a reference dimension. In some embodiments, an operator (e.g., a doctor) may directly input the one or more key positions via a terminal device (e.g., the terminal device 140). For example, if the subject is a patient, the operator may designate the hands, shoulders, waist, pelvis, etc., as the one or more key positions. In some embodiments, the one or more key positions may be default settings of the scanning system 100. For example, different types (e.g., upper torso, lower torso, etc.) of subjects may correspond to different key positions. Before scanning the subject, the operator may input the type of the subject, the processing device 120 may determine the one or more key positions based on the input type of the subject. Further, the processing device 120 may obtain the reference dimensions of the subject along the width direction of the table at the one or more key positions using an imaging device (e.g., a CT device) or a camera. For example, the imaging device (e.g., a CT device) or the camera may acquire an image of the subject, and determine the reference dimensions by measuring dimensions of the subject at the one or more key positions based on the image of the subject.

In some embodiments, the operator may directly input the reference dimensions, along the width direction of the table, of the subject on the table at the positions along the moving direction of the table.

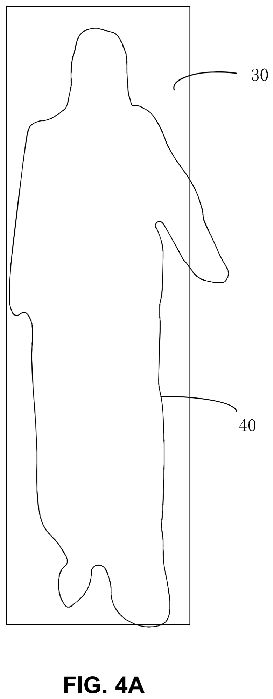

In some embodiments, the processing device 120 may determine the reference dimensions based on a contour of the subject. The contour refers to edge lines of the subject's external form, which does not include internal details such as textures. FIG. 4A shows an exemplary contour 40 of a subject on a table according to some embodiments of the present disclosure.

In some embodiments, the contour of the subject may be the entire body contour of the subject or a portion contour of the subject. For example, as shown in FIG. 4A, the contour 40 is the entire body contour of the subject. As another example, the contour may be an upper body contour of the subject. The present disclosure may take obtaining the subject's entire body contour as an example for illustration.

In some embodiments, the processing device 120 may obtain the contour of the subject based on contour information (i.e., information related to the contour) of the subject. In some embodiments, the contour information may include an image (e.g., an optical image or a medical image) of the subject lied on the table. The processing device 120 may obtain the image of the subject using an imaging device (e.g., a CT device) or a camera. For example, a camera may be arranged above the table, e.g., on a gantry of the SPECT device or on the ceiling of the room in which the SPECT device is located. After the subject lies on the table, the camera may capture an image of the subject. The processing device 120 may process the image (i.e., the contour information) to obtain the contour of the subject. For example, the processing device 120 may perform grayscale processing on the image and finally extract the subject's contour edges using edge detection algorithms. Exemplary edge detection algorithms may include a gradient-based algorithm, a Laplacian-based algorithm, a deep learning-based edge detection algorithm, etc. As another example, after the subject lies on the table, the processing device 120 may obtain an image of the subject using an imaging device (e.g., a CT device) other than the SPECT device to scan the subject. Then, the processing device 120 may process the image (i.e., the contour information) to obtain the contour using, e.g., an edge detection algorithm.

In some embodiments, the processing device 120 may directly obtain the contour of the subject. For example, a large number of pressure sensors may be embedded in the table. When the subject lies on the table, the large number of pressure sensors may form a two-dimensional human body contour distribution based on a pressure-bearing status (e.g., regions where the pressure value exceeds a pressure threshold are determined as human body-covered regions), which can be then mapped to a scanning coordinate system through coordinate transformation to generate the contour of the subject. As another example, the processing device 120 may obtain the contour of the subject from a contour database based on feature information of the subject. The feature information of the subject may include, but is not limited to, the gender, age, height, weight, etc. The contour database includes a correspondence between the feature information of the subject and the contour of the subject. The contour database may be pre-constructed based a large number of people's real contours and their feature information.

After the contour of the subject is obtained, the processing device 120 may determine the reference dimensions based on the contour of the subject. For example, the processing device 120 may designate dimensions of the contour at the one or more key positions along the width direction of the table as the reference dimensions of the subject.

As another example, the processing device 120 may divide the contour along the moving direction of the table into a threshold count (e.g., 2, 3, 4, 5, etc.) of contour parts. In some embodiments, the processing device 120 may divide the contour along the moving direction of the table based on a length of the contour along the moving direction of the table and a preset scanning size of the detector along the moving direction of the table. As used herein, the preset scanning size of the detector refers to a maximum spatial range along the moving direction of the table that the detector can cover during a single scan. For example, the processing device 120 may determine a quotient by dividing the length of the contour by the preset scanning size. Further, the processing device 120 may divide the contour along the moving direction of the table based on the quotient. For example, when the quotient is an integer, the processing device 120 may divide the contour along the moving direction of the table such that a count of contour parts is equal to the quotient and a size of each contour part along the moving direction of the table is equal to the preset scanning size. As another example, when the quotient is not an integer, the processing device 120 may determine an integer greater than the quotient as the count of contour parts, and divide the contour along the moving direction of the table such that the size of each contour part along the moving direction of the table, except for the last contour part, is equal to the preset scanning size of the detector. Further, the processing device 120 may determine a dimension, along the moving direction of the table, at a position corresponding to a connection point of any two adjacent contour parts as the reference dimension of the subject. According to some embodiments of the present disclosure, by determining the reference dimensions of subject based on the contour of the subject, one can focus only the subject's external edge lines, and directly lock the leftmost and rightmost key positions of the subject, avoiding width measurement deviations caused by interferences (such as clothing texture, wrinkles, accessories, etc), making the determination process fast, accurate, and anti-interference.

In 320, the processing device 120 (e.g., the region division module 220) may divide, based on the reference dimensions, the subject into at least one region along the moving direction of the table.

In some embodiments, the at least one region may include at least one of a first region or a second region. The first region may include a region where a dimension of the subject along the width direction of the table is smaller than or equal to a preset scanning width of the detector along the width direction of the table. As used herein, the preset scanning width of the detector refers to a maximum spatial range along the width direction of the table that the detector can cover during a single scan. In other words, a preset scanning range of the detector along the width direction of the table can cover the first region. In some embodiments, the first region may also be referred to as a fully-covered region or a full FOV region. The second region may include a region where a dimension of the subject along the width direction of the table is greater than the preset scanning width of the detector along the width direction of the table. In other words, the preset scanning range of the detector along the width direction of the table cannot cover the second region. In some embodiments, the second region may also be referred to as a partially-covered region or a truncated FOV region.

As used herein, a dimension of the subject in a region (e.g., the first region or the second region) may refer to a maximum dimension of the subject along the width direction of the table in that region.

In some embodiments, the processing device 120 may divide the subject into a plurality of regions based on the reference dimensions. For example, the processing device 120 may determine a part between positions corresponding to two adjacent reference dimensions as a region, or determine a part between a position corresponding to a reference dimension and a starting/ending position of the subject along the moving direction of the table as a region. In such cases, a count of the plurality of regions may be one more than a count of the reference dimensions. As another example, the processing device 120 may determine positions, each of which is between two positions corresponding to two adjacent reference dimensions, as target positions. The processing device 120 may determine a part between any two adjacent target positions as a region, or divide a part between a position corresponding to a target position and the starting/ending position of the subject along the moving direction of the table as a region. In such cases, a count of the plurality of regions may be equal to a count of the reference dimensions.

It should also be noted that in some embodiments, the processing device 120 may divide the subject into the plurality of regions based on contour of the subject. For example, the processing device 120 may determine a quotient by dividing the length of the contour along the moving direction of the table by the preset scanning size of the detector along the moving direction of the table. Further, the processing device 120 may divide the subject into the plurality of regions based on the quotient. For example, the processing device 120 may determine the smallest integer greater than or equal to the quotient as a count of the plurality of regions, and divide the subject along the moving direction of the table such that the size of each region along the moving direction of the table, except for the last region, is equal to the preset scanning size of the detector.

The processing device 120 may determine a maximum dimension of each region along the width direction of the table. The processing device 120 may designate each region as a first region or a second region based on the maximum dimension of the region along the width direction of the table. For example, the processing device 120 may compare the maximum dimension of each region with the preset scanning width of the detector along the width direction of the table. If the maximum dimension of a region is smaller than or equal to the preset scanning width of the detector along the width direction of the table, the processing device 120 may designate that region as a first region. If the maximum dimension of a region is greater than the preset scanning width of the detector along the width direction of the table, the processing device 120 may designate that region as a second region.

In 330, the processing device 120 (e.g., the control module 330) may control a relative motion between the detector and the table to perform scanning on the at least one region in sequence along the moving direction of the table.

In some embodiments, for scanning the first region, since the preset scanning range of the detector along the width direction of the table can cover the first region, and the movement of the gantry is relatively complex, while the movement of the table is relatively simple, in order to simplify the movement of the SPECT device (or the scanning system 100), the processing device 120 may control the movement of the table to achieve the relative motion. In such cases, both the detector and the gantry may remain stationary, while the table with the subject is controlled to move along the moving direction (or the length direction) of the table to allow the relative motion between the detector and the table, enabling scanning of the first region of the subject along the moving direction of the table.

In some embodiments, for scanning the second region, since the preset scanning range of the detector along the width direction of the table cannot cover the second region, the processing device 120 may control a movement of the detector to achieve the relative motion. In such cases, the table with the subject may remain stationary, while the detector is controlled to move along the width direction of the table to complete the scan of the second region of the subject. For example, the second region may have a first end and a second end along the width direction of the table. The processing device 120 may control the detector to move along the width direction of the table from one of the first end and the second end to the other of the first end and the second end. It should be noted that in the present disclosure, since the detector has a certain size, i.e., the detector may have a scanning range (or a scan field of view) at each position of the detector, controlling/causing the detector to move from an end of a first reference region to an end of a second reference region may refer to controlling the preset scanning range of the detector from covering at least the end of the first reference region to covering at least the end of the second reference region. The first reference region may be the same as or different from the second reference region. For example, the first reference region may be a first region, and the second reference region may be a second region. As another example, the first reference region may be a second region, and the second reference region may be a first region. Therefore, when scanning a subject (such as an obese individual) having an ultra-width, the subject may not need to turn to his/her left and right sides separately for rescanning, improving the comfort of the subject and the scanning efficiency.

It should be noted that in some embodiments of the present disclosure, the relative motion between the detector and the table achieved by controlling the table to move may also be achieved by controlling the gantry or the detector to move, or the relative motion between the detector and the table achieved by controlling the detector to move may also be achieved by controlling the table to move, which will not elaborate further. Merely by way of example, the relative motion between the detector and the table along the width direction of the table may be achieved by controlling the detector to be stationary, while controlling the table to move along the width direction of the table. Unless otherwise specified, the moving direction of the table described in the present disclosure refers to the length direction of the table. It should also be noted that, during the process for scanning the first region, the table may move in a step-and-shoot manner or a uniform continuous manner, while during scanning the second region, the table may move in a step-and-shoot manner. As used herein, the step-and-shoot manner refers to a moving manner in which the table moves in a preset fixed step (usually relating to the detector's axial field of view). After each movement, the table may stop completely until the detector completes data acquisition. Once the data acquisition ends, the table may move in the preset fixed step again. The uniform continuous manner refers to a moving manner in which the table passes through a detecting region of the gantry at a constant speed (e.g., 0.3 mm/s-1 mm/s).

In some embodiments, if a size of the second region along the moving direction of the table is greater than the preset scanning size of the detector, the second region may be divided into a plurality of sub-regions along the moving direction of the table. In some embodiments, the processing device 120 may divide the size of the second region along the moving direction of the table by the preset scanning size of the detector along the moving direction of the table to obtain a count of sub-regions. Thus, the second region may be divided into sub-regions of that count. In some embodiments, the processing device 120 may evenly divide the second region into the sub-regions of that count. In some embodiments, for the convenience of data processing, the processing device 120 may determine sizes, along the moving direction of the table, of sub-regions other than the last sub-region to be equal to the preset scanning size of the detector along the moving direction of the table, and determine a size of the last sub-region to be equal to a difference between the size of the second region along the moving direction of the table and a sum of the sizes of the sub-regions other than the last sub-region along the moving direction of the table.

In some embodiments, the second region may include a first sub-region and a second sub-region adjacent to each other along the moving direction of the table. Each of the first sub-region and the second sub-region may have a first end and a second end along the width direction of the table. The first end of the first sub-region and the first end of the second sub-region may be located on the same side of the subject. In such cases, the processing device 120 may control the detector to move from the first end of the first sub-region to the second end of the first sub-region to scan the first sub-region. The processing device 120 may cause the detector to move from the second end of the first sub-region to the second end of the second sub-region. For example, the processing device 120 may control the detector to move along the moving direction of the table from the second end of the first sub-region to the second end of the second sub-region. In some embodiments, the processing device 120 may control the table to move along its moving direction to enable the detector to move from the second end of the first sub-region to the second end of the second sub-region. Further, the processing device 120 may control the detector to move from the second end of the second sub-region to the first end of the second sub-region to scan the second sub-region. In some embodiments, the above-mentioned scanning manner for scanning the second region having the plurality of sub-regions along the moving direction of the table may be referred to as an "S-shaped" scanning manner.

In some embodiments, if a size of the second region along the moving direction of the table is smaller than or equal to the preset scanning size of the detector, each second region among two or more consecutive second regions may be regared as a sub-region as described above. The processing device 120 may control the SPECT device to scan the two or more consecutive second regions in a manner similar to scanning the second region having a plurality of sub-regions.

According to some embodiments of the present disclosure, by using the aforementioned scanning path to scan the second region, which has the shortest motion path of the detector, the scanning time can be shortened, thereby improving the scanning efficiency.

In some embodiments, the first region and the second region may be arranged in sequence along the moving direction of the table. In other words, the detector may need to scan the first region first and then scan the second region. In such cases, for scanning the first region, the table may not need to move along the OX direction, and the processing device 120 may merely control the table to move along or off a central axis of a bore formed by the gantry to scan the first region. For example, the table may move along a specific axis where a specific position (e.g., a central position) of the gantry in the width direction of the table is located. The specific position is a position of the detector on the gantry, as long as the preset scanning range of the detector along the width direction (i.e., the OX direction) of the table at that position can cover the first region. The specific axis is parallel to the central axis or the moving direction of the table. It should be noted that in some embodiments, when a size of the first region along the moving direction of the table is less than or equal to the preset scanning size of the detector, once the scanning range of the detector covers the first region, the processing device 120 may control the table to stop moving. Then, the processing device 120 may control the table to move along the specific axis until a scanning range of the detector transitions from the first region to the second region. An axis parallel to the moving direction of the table where the central position of the gantry in the width direction of the table is located also refers to a central axis. It should be noted that since the detector has a certain size, i.e., the detector may have a scanning range at each position of the detector, the transition of the detector's scanning range from a first reference region to a second reference region may refer to the detector changings from scanning at least a portion of the first reference region to scanning a portion that fully belongs to the second reference region. The first reference region may be different from the second reference region. For example, the first reference region may be a first region, and the second reference region may be a second region. As another example, the first reference region may be a second region, and the second reference region may be a first region.

In some embodiments, after the detector completes scanning the first region, the processing device 120 may control the table to move such that a scanning range of the detector transitions from the first region to the second region. The processing device 120 may control the detector to move from the specific position (e.g., the central position) of the gantry to a first end of the second region. The processing device 120 may control the detector to move from the first end of the second region to a second end of the second region to scan the second region. In order to save scanning time and energy, a distance between the first end and the specific position (e.g., the central position) may be less than or equal to a distance between the second end and the specific position (e.g., the central position).

In some embodiments, after the detector completes scanning the second region, the processing device 120 may control the detector to move back to the specific position (e.g., the central position) of the gantry. The processing device 120 may control the table to move to scan a next first region (i.e., a next fully-covered region).

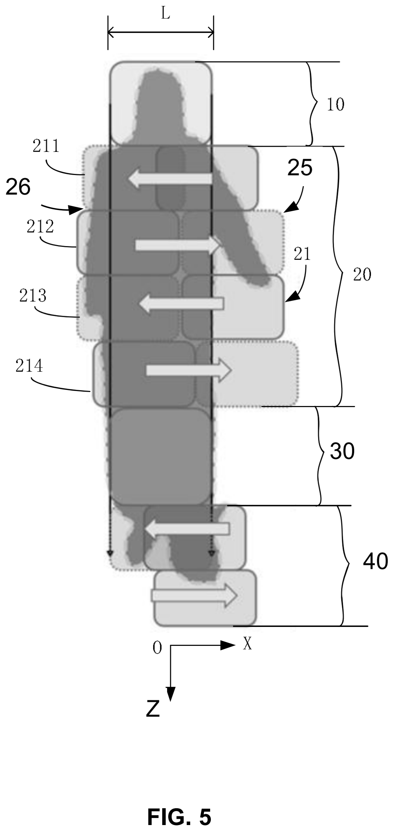

More descriptions for scanning the first region or the second region may be found elsewhere in the present disclosure, e.g., FIG. 5 and the descriptions thereof.

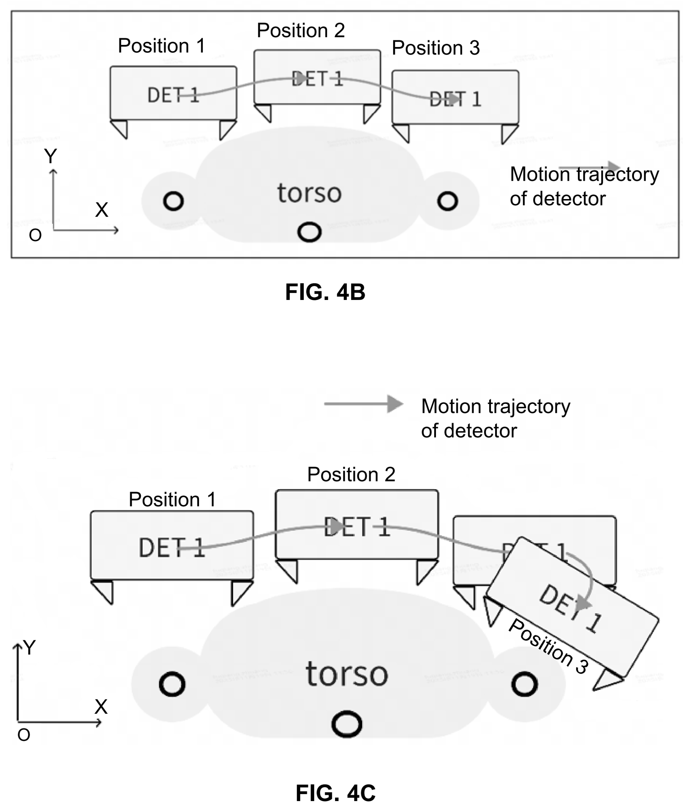

In some embodiments, in order to obtain a high-resolution image of the subject, the detector should be as close to the subject as possible for scanning. However, due to the respiratory motion of the human body, the surface of the human body along the OY direction as shown in 4B exhibits undulations. Therefore, for scanning the second region, when the detector moves along the width direction of the table, the detector may need to move synchronously in the OY direction to avoid collision with the subject, such that a smallest distance between the detector and the subject (or the surface of the subject) can be within a distance range. The distance range may be set based on experience, which allows the detector to be as close to the subject as possible and less likely to collide with the subject. For example, FIG. 4B is a schematic diagram illustrating an exemplary moving process of a detector according to some embodiments of the present disclosure. As shown in FIG. 4B, at the beginning for scanning the second region along the width direction of the table (i.e., the OX direction), the detector (DET 1) may be loated at position 1 that is relatively close to the table. Then, due to the respiratory motion of the subject, the detcector (DET 1) may move to position 2. Position 2 is farther from the table than position 1 along the OY direction. During the moving process of the detector from position 1 to position 2, the detector (DET 1) may move synchronously in the OX direction and the OY direction. Finally, the detector (DET 1) may move to position 3 that is relatively close to the table. A distance from position 1 and position 3 to the table may be the same or different. During the moving process of the detector from position 2 to position 3, the detector (DET 1) may also move synchronously in the OX direction and the OY direction.

In some embodiments, in order to further improve the resolution of the final reconstructed image, during the moving process of the detector along the width direction of the table, the detector may not noly move synchronously in the OX direction and the OY direction but also rotate around its own axis, allowing the detector to be as close as possible to the subject for scanning. For example, FIG. 4C is a schematic diagram illustrating an exemplary moving process of a detector according to some embodiments of the present disclosure. As shown in FIG. 4C, during the moving process of the detector from position 2 to position 3, unlike what is shown in FIG. 4B, the detector (DET 1) may not only move synchronously in the OX direction and the OY direction, but also rotate around its own axis. Correspondingly, an image of the subject may be reconstructed based on raw imaging data and rotation angle information of the detector.

In some embodiments, the processing device 120 may automatically control the detector to avoid the collision between the detector and the subject based on depth information between the detector and the subject (or the surface of the subject). In some embodiments, the depth information between the detector and the subject may be acquired using a plurality of sensors. Each sensor may acquire reference depth information between a position where the sensor is installed and the subject. The processing device 120 may determine the depth information between the detector and the subject based on the reference depth information and a position of the detector. In some embodiments, the sensor(s) may include a ultrasonic radar, a single-line/multi-line LiDAR, a structured light sensor, an infrared depth camera, or the like, or any combination thereof.

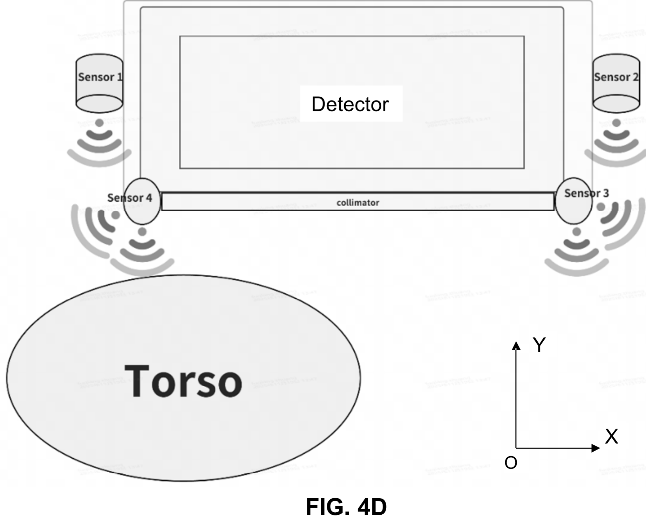

In some embodiments, the sensor(s) may be directly instaled on the detector. Thus, the reference depth information acquired by the sensor(s) may be the depth information between the detector and the subject. For example, FIG. 4D shows exemplary sensors on a detector according to some embodiments of the present disclosure. As shown in FIG. 4D, sensor 1, sensor 2, sensor 3, and sensor 4 are installed at different positions of the detector. These sensors may obtain the depth information between the detector and the subject directly based on e.g., time of flight (TOF), a time difference between signal transmission and reception, etc.

In some embodiments, the sensor(s) may be not instaled on the detector. For example, the sensor(s) may be an infrared depth camera arranged above the table, e.g., on the gantry of the SPECT device or on the ceiling of the room in which the SPECT device is located. It should be noted that in some embodiments, the aforementioned camera used to obtain the contour of the subject may also be used to obtain the depth information between the detector and the subject, thus reducing the installation of hardware configurations and saving costs. In some embodiments, the aforementioned imaging device (e.g., a CT device) used to obtain the contour of the subject may also be used to obtain the depth information between the detector and the subject.

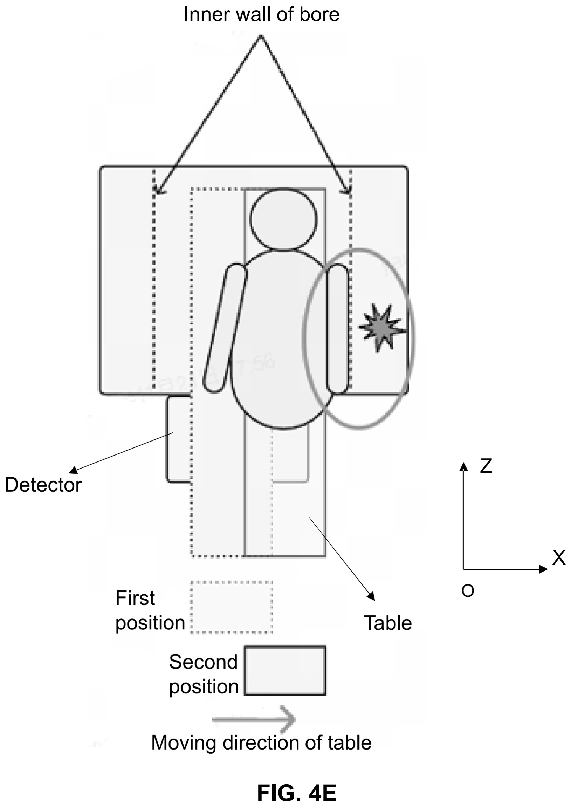

It should be noted that in some embodiments, when the relative motion between the detector and the table along the width direction of the table is achieved by controlling the detector to be stationary and controlling the table to move along the width direction of the table, the processing device 120 may control the table based on depth information between the SPECT device and the subject (or the surface of the subject) to avoid collision between the SPECT device (e.g., an inner wall of a bore formed by the gantry) and the subject. The control manner is similar to that for avoiding collision between the detector and the subject, and will not be repeated herein. In some embodiments, one or more collision sensors may be arrange at the inner wall of the bore to avoid the risk of collision between the subject and the inner wall of the bore (such as the scanned patient suddenly stretches out his/her hand, resulting in his/her body exceeding the previously acquired contour range, etc.). In some embodiments, the collision sensor may be a membrane switch, which is a type of sensor that changes its surface resistance value when compressed. In some embodiments, the collision sensor may be a pressure sensor connected to the inner wall of the bore to detect whether there is overall offset of a cylinder where the inner wall is located. For example, FIG. 4E is a schematic diagram illustrating collision between a subject and an inner wall of a bore of an SPECT device according to some embodiments of the present disclosure. As shown in FIG. 4E, during the scanning the second region of the subject, when the table moves along the OX direction, the subject may collide with the inner wall of the bore. Thus, by using the collision sensors, in response to the contact between the subject and the inner wall of the bore, the processing device 120 may immediately control the table to stop, thereby avoiding harm to the subject.

In some embodiments, the processing device 120 may control the detector and the table to perform the scanning on the at least one region based on a target motion parameter set. The target motion parameter set may include motion parameters of the detector and the table at each time point, that is, during the process for scanning the at least one region, the processing device 120 may control the detector to move along a movement path of the detector and/or control the table to move along a movement path of the table based on the target motion parameter set. In some embodiments, the motion parameters may include a position, a speed, an acceleration, etc., of the table and/or a position, a speed, an acceleration, etc., of the detector at each time point. It should be noted that the time points during the scanning process of the subject may be at a millisecond level or even a smaller level. In other words, a scanning time of the subject may be divided into a plurality of time points at a millisecond level or even a smaller level.

In some embodiments, the processing device 120 may determine the target motion parameter set by looking up a motion parameter set library based on a dimension of the subject along the width direction of the table in each region of the at least one region.

Merely by way of example, before the scanning system 100 leaves the factory, engineers may pre-design and store a variety of motion parameter set templates for different sample regions. For example, a first motion parameter set template corresponding to a time period for scanning a sample first region may be set so that a result is as follows: the table moves longitudinally (i.e., along the length direction of the table) at a constant speed $V_{table}$, while the detector remains stationary. The sample first region may include a region where a dimension of a sample subject along the width direction of the table is smaller than or equal to the preset scanning width of the detector along the width direction of the table. A total time $T_1$ for scanning the sample first region may be calculated according to formula (1) as follows:

$$T_1 = \frac{L_1}{V_{table}}, \quad (1)$$

where $L_1$ denotes a size of the sample first region along the length direction of the table.

As another example, a second motion parameter set template corresponding to a time period for scanning a sample second region may be set so that a result is as follows: a scanning manner of the sample second region is the 'S-shaped' scanning manner as described above. The sample second region may include a region where a dimension of a sample subject along the width direction of the table is greater than the preset scanning width of the detector along the width direction of the table. A total time $T_2$ for scanning the sample second region may be calculated according to formula (2) as follows:

$$T_2 = \frac{W_2}{V_{detector}} \times N + (N-1) \times \frac{L_{step1}}{V_{step}}, \quad (2)$$

where $W_2$ denotes a size of a sample sub-region along the width direction of the table, $V_{detector}$ denotes a moving speed of the detector in the width direction of the table, N denotes a count of sample sub-regions into which the sample second region is divided, $L_{step1}$ denotes a step distance of the table during the transition of detector's scanning range from a sub-region to an adjacent sub-region, and $V_{step}$ denotes a step speed of the table.

As a further example, a third motion parameter set template correspongding to a time period for controlling a scanning range of the detector to transition from the sample first region to the sample second region may be set so that a result is as follows: the table is controlled to move along a sample specific position (e.g., the central position), in the width direction of the table, of the gantry carrying the detector until a scanning range of the detector transitions from a sample first region to an adjacent sample second region. A total time $T_3$ for controlling the scanning range of the detector to transition from the sample first region to the sample second region may be calculated according to formula (3) as follows:

$$T_3 = \frac{L_{T1}}{V_0} + \frac{L_3}{V_{detector'}}, \quad (3)$$

where $L_{T1}$ denotes a moving distance of the table during a process for controlling a scanning range of the detector to transition from the sample first region to the sample second region, $V_0$ denotes a moving speed of the table during the process for controlling the scanning range of the detector to transition from the sample first region to the sample second region, $L_3$ denotes a distance between the sample specific position and a specific end of the second sample region that is closer to the sample specific position than the other end of the second sample region, and $V_{detector'}$ denotes a moving speed of the detector when the detector moves from the sample specific position to the specific end of the second sample region.

As another further example, a fourth motion parameter set template correspongding to a time period for controlling a scanning range of the detector to transition from the sample second region to the sample first region may be set so that a result is as follows: the detector is controlled to move back to the sample specific position of the gantry, and the table is controlled to move until a scanning range of the detector transitions from the sample second region to the sample first region. A total time $T_4$ for controlling the scanning range of the detector to transition from the sample second region to the sample first region may be calculated according to formula (4) as follows:

$$T_4 = \frac{L_4}{V_{detector'}} + \frac{L_{T2}}{V_0'}, \quad (4)$$

where $L_4$ denotes a distance between a scan ending end of the second sample region and the sample specific position, $V_{detector'}$ denotes the moving speed of the detector when the detector moves from the scan ending end of the sample second region to the sample specific position, $L_{T2}$ denotes a moving distance of the table during a process for controlling a scanning range of the detector to transition from the sample second region to the sample first region, $V_0'$ denotes a moving speed of the table during the process for controlling the scanning range of the detector to transition from the sample second region to the sample first region .

The processing device 120 may match each region with an optimal motion parameter set template in the motion parameter set template library based on the dimension of the subject along the width direction of the table of each region in the at least one region. For example, for a certain region, if a dimension of the certain region of the subject along the width direction of the table is smaller than or equal to the preset scanning width of the detector along the width direction of the table, the processing device 120 may determine the first motion parameter set template is the optimal motion parameter set template of the certain region. If the dimension of the certain region of the subject along the width direction of the table is greater than the preset scanning width of the detector along the width direction of the table, the processing device 120 may determine the second motion parameter set template is the optimal motion parameter set template of the certain region. The processing device 120 may further determine an optimal motion parameter set template between any two adjacent different regions.

The processing device 120 may obtain geometric dimensions (e.g., a length and width of each region, a distance between the specific position and a specific end of a second region that is closer to the specific position, a distance between a scan ending end of a second region and the specific position) associated with the at least on region, and substitute these specific geometric dimensions into the formula corresponding to the matched optimal motion parameter set template to calculate specific motion parameters of each time period at each time point. The processing device 120 may splice the motion parameters of all time periods for scanning the at least one region in a scanning order (e.g., from the head to feet of the subject, or from the feet to head of the subject, or along an axial direction of the subject) to form the target motion parameter set that covers the entire scanning process. According to some embodiments of the present disclosure, by controlling the detector and the table to perform the scanning based on the target motion parameter set, the automation and execution accuracy of the scanning process are greatly improved, not only optimizing scanning efficiency, but also providing a solid data acquisition foundation for the obtained image, thereby significantly enhancing the scanning stability and reliability of the scanning system 100.

In some embodiments, the processing device 120 may determine the target motion parameter set based on a constraint condition and an optimization objective. The constraint condition may include a coverage ratio of a total scanning area to an area of the subject being greater than 100%, i.e., the whole subject needs to be scanned. In some embodiments, the constraint condition may also include a maximum speed constraint, an anti-collision constraint, a maximum acceleration constraint, or the like, or any combination thereof. As used herein, the maximum speed constraint refers that a motor speed of the SPECT device not exceeds a rated speed of the motor. The anti-collision constraint refers to a constraint in which the detector does not collide with the subject or other components of the SPECT device. The maximum acceleration constraint refers that an acceleration of the motor of the SPECT device not exceeds a rated acceleration of the motor, so as to avoid mechanical damage to the subject or the SPECT device itself.

The optimization objective may include minimizing a total scanning time. In some embodiments, the optimization objective may further include an image quality optimization objective, a mechanical loss optimization objective, a motion smoothness optimization objective, or the like, or any combination thereof. As used herein, the image quality optimization objective may include maximizing the image quality of a finally obtained image. The mechanical loss optimization objective may include minimizing the mechanical loss of the SPECT device. The motion smoothness optimization objective may include maximizing the motion smoothness degree of the table or the detector.

The processing device 120 may determine the motion parameter set using various optimization algorithms based on the constraint condition and the optimization objective. Merely by way of example, the processing device 120 may virtually divide the subject into a two-dimensional grid composed of a large number of tiny squares (e.g., 1cm x 1cm) to obtain a grid map. Each square represents a node that must be scanned. The processing device 120 may assign a cost value to each possible edge (i.e., an action of moving the detector or the table from one node to an adjacent node) in the grid map. The cost value may be calculated based on the optimization objective. For example, if the optimization objective is minimizing the total scanning time, then the cost value may be equal to a time required to move. As another example, if the optimization objective further includes optimizing the image quality, a weight may be added to the cost value of the edges between nodes in a region of interest (ROI), which can induce the optimization algorithm to stay a little longer in the ROI (i.e., reduce speed). As a further example, if the optimization objective further includes minimizing a mechanical loss of the SPECT device, a penalty weight may also be added to the cost value of edges representing sharp turns or high acceleration.

Further, the processing device 120 may determine the constraint condition being that a scanning trajectory for scanning the subject is set such that all nodes in the grid map are visited at least once. In some embodiments, the constraint condition may also include the maximum speed constraint to limit the motor speed of the SPECT device from exceeding the rated speed of the motor. Taking a scanning starting point as an initial node of the grid map the processing device 120 may run a graph search algorithm (e.g., an A* algorithm or its variants, a genetic algorithm, etc.) to obtain a minimum cost path, i.e., an optimal scanning trajectory. The processing device 120 may convert the optimal scanning trajectory into precise motion parameters (i.e., the target motion parameter set) containing velocity and acceleration information based on its corresponding parameter control. According to some embodiments of the present disclosure, by determining the target motion parameter set based on the constraint condition and the optimization objective, the planning of scanning path is upgraded from "experience driven" to "data-driven intelligent decision-making," ensuring the completeness and accuracy of scanning, fundamentally eliminating the risk of missed scans. Moreover, with the optimization goal of "total scanning time", the SPECT device can automatically find and execute the shortest path among countless possible scanning paths through algorithms, which can maximize the operational efficiency of the SPECT device, shorten patient waiting time, and bring significant economic and management benefits to medical institutions while ensuring diagnostic quality.

In some embodiments, the processing device 120 may update the target motion parameter set based on a movement of the subject. For example, during a scanning process, the processing device 120 may monitor the movement of the subject at each time point using, e.g., a camera. In response to determining the movement of the subject satisfying a movement condition, the processing device 120 may pause the current relative motion between the detector and the table immediately. The movement condition may include the subject moving a distance greater than a threshold. For example, the subject moves 2 cm to the left. The processing device 120 may obtain a new position of the subject on the table, and update a relative position between the detector and the table to allow the scanning range of the detector can cover the subject when controlling the relative motion between the detector and the table. The processing device 120 may determine an updated target motion parameter set by re-running the optimization algorithm based on the new position of the subject, and control the detector to scan the remaining unscanned regions based on the updated target motion parameter set.

According to some embodiments of the present disclosure, by updating the target target motion parameter set in responose to the movement of the subject, the remaining unscanned regions can be fully scanned, ensuring that the finally obtained image quality is not affected. This enhances the robustness of the scanning process and its adaptability to real clinical environments. It not only avoids examination failures and expensive rescanning caused by the subject's movement, but also ensures the success rate of data collection under complex conditions, significantly enhancing the clinical practical value of the scanning system 100.

It should be noted that the above description is merely provided for the purposes of illustration, and is not intended to limit the scope of the present disclosure. For persons having ordinary skills in the art, multiple variations and modifications may be made under the teachings of the present disclosure. However, those variations and modifications do not depart from the scope of the present disclosure. In some embodiments, one or more operations may be omitted and/or one or more additional operations may be added. For example, operation 310 and operation 320 may be combined into a single operation. As another example, one or more other optional operations (e.g., a reconstruction operation) may be added. For example, after the detector completes scanning the at least one region, the processing device 120 may further reconstruct an image of the subject based on the obtained scanning data.

FIG. 5 is a schematic diagram illustrating a process for scanning a subject having a plurality of regions along a moving direction of a table according to some embodiments of the present disclosure. As shown in FIG. 5, the subject may be divided into four regions including two first regions 10 and 30 and two second regions 20 and 40. The two first regions 10 and 30 and two second regions 20 and 40 may be arranged alternately.

When scanning the subject, the subject may lie on a table of a SPECT device. The SPECT device (or a detector of the SPECT) may scan the subject from his/her head to feet (or from his/her feet to head) to obtain a complete image of the subject. For ease of description, the present disclosure will use scanning the head first as an example.

In some embodiments, for scanning the first region 10 (e.g., the head), the detector of the SPECT device may be stationary at a specific position of a gantry carrying the detector, as long as a preset scanning range of the detector along a width direction (i.e., the OX direction shown in FIG. 5) of the table can cover the first region 10. For example, the detector may be stationary at a central position, in the width direction of the table, of the gantry, which is conducive to coinciding with the midline (or an axial direction) of the subject's contour in the width direction of the table, facilitating covering the first region 10 along the width direction of the table. A relative motion between the detector and the table along a moving direction (i.e., the OZ direction shown in FIG. 5) of the table may be achieved, so that the detector can scan the subject (the first region 10) along the moving direction of the table.

After the detector completes scanning the first region 10, the table may be controlled to move such that a scanning range of the detector transitions from the first region 10 to the second region 20.

The second region 20 may have a plurality of sub-regions 21. For example, as shown in FIG. 5, the second region 20 may have a first sub-region 211, a second sub-region 212, a third sub-region 213, and a fourth sub-region 214. Each sub-region may have a first end 25 and a second end 26 along the width direction (i.e., the OX direction) of the table. The first ends 25 of the plurality of sub-regions may be located on a same side of the subject. In some embodiments, a size of the first sub-region 211, the second sub-region 212, or the third sub-region 213 along the OZ direction may be equal to a preset scanning size of the detector along the OZ direction. A size of the fourth sub-region 214 may be smaller than or equal to the preset scanning size of the detector along the OZ direction. For example, when the size of the second region 20 along the OZ direction cannot be divided evenly by the preset scanning size of the detector along the OZ direction, the size of the last sub-region (i.e., the fourth sub-region 214) of the plurality of sub-regions along the OZ direction may be smaller than the preset scanning size of the detector along the OZ direction. When the size of the second region 20 along the OZ direction can be divided evenly by the preset scanning size of the detector along the OZ direction, the size of the last sub-region (i.e., the fourth sub-region 214) of the plurality of sub-regions along the OZ direction may equal to the preset scanning size of the detector along the OZ direction.

For scanning the first sub-region 211, the detector may firstly be controlled to move from the specific position (e.g., the central position) of the gantry to the first end 25 or the second end 26 of the second region 20. In some embodiments, in order to save scanning time and energy, an end of the second region 20 with a shorter distance from the detector (i.e., the specific position) along the OX direction may be selected. For ease of description, the first end 25 may be taken as an example, i.e., the detector may first be controlled to move from the specific position to the first end 25. The detector may be controlled to move from the first end 25 of the first sub-region 211 to the second end 26 of the first sub-region 211 to scan the first sub-region 211.

After the detector completes scanning the first sub-region 211, the detector may be caused to move from the second end 26 of the first sub-region 211 to the second end 26 of the second sub-region 212. For example, the table may be controlled to move along the OZ direction, or the detector may be controlled to move along the OZ direction, so that the detector can scan the second sub-region 212. Then, the detector may be controlled to move from the second end 26 of the second sub-region 212 to the first end 25 of the second sub-region 212 to scan the second sub-region 212.

Similarly, after the detector completes scanning the second sub-region 212, the detector may be caused to move from the first end 25 of the second sub-region 212 to the first end 25 of the third sub-region 213. The detector may be controlled to move from the first end 25 of the third sub-region 213 to the second end 26 of the third sub-region 213 to scan the third sub-region 213. After the detector completes scanning the third sub-region 213, the detector may be caused to move from the second end 26 of the third sub-region 213 to the second end 26 of the fourth sub-region 214. The detector may be controlled to move from the second end 26 of the fourth sub-region 214 to the first end 25 of the fourth sub-region 214 to scan the fourth sub-region 214.

According to some embodiments of the present disclosure, by using the aforementioned scanning path to scan the second region 20, which has the shortest motion path of the detector, the scanning time can be shortened, thereby improving the scanning efficiency.

After the detector completes scanning the second region 20 (i.e., the fourth sub-region 214), the table may be controlled to move such that a scanning range of the detector transitions from the second region 20 to the first region 30. The detector may be controlled to move to a specific position of the gantry, as long as the preset scanning range of the detector along the OX direction can cover the first region 30. For example, the detector may be controlled to move back to the specific position (e.g., the central position) of the gantry. Further, the table may be controlled to move along the OZ direction to enable the detector to scan the first region 30.

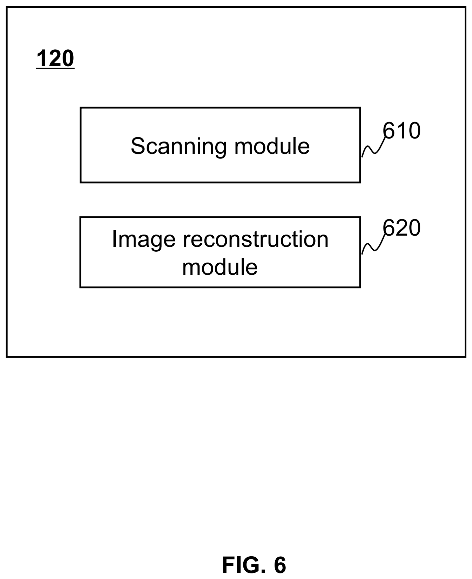

FIG. 6 is a block diagram illustrating an exemplary processing device according to some embodiments of the present disclosure. As illustrated in FIG. 6, the processing device 120 may include a scanning module 610 and an image reconstruction module 620. Each of the modules described above may be a hardware circuit that is designed to perform certain actions, e.g., according to a set of instructions stored in one or more storage media, and/or any combination of the hardware circuit and the one or more storage media.

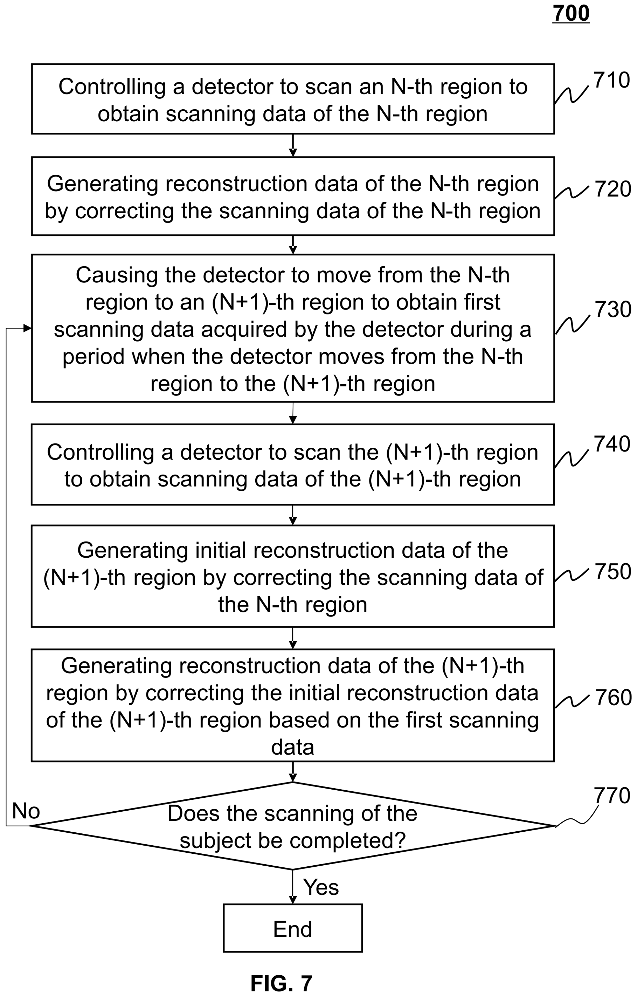

The scanning module 610 may be configured to control a detector to scan an N-th region to obtain scanning data of the N-th region. N may be a positive integer greater than 1. In some embodiments, the scanning module 610 may also cause the detector to move from the N-th region to an (N+1)-th region to obtain first scanning data acquired by the detector during a period when the detector moves from the N-th region to the (N+1)-th region. The scanning module 610 may further control the detector to scan the (N+1)-th region to obtain scanning data of the (N+1)-th region. More descriptions regarding the scanning a region of the subject may be found elsewhere in the present disclosure, e.g., FIG. 3 and FIG. 7 and relevant descriptions thereof.

The image reconstruction module 620 may be configured to generate reconstruction data of the N-th region by correcting the scanning data of the N-th region. In some embodiments, the image reconstruction module 620 may also generate initial reconstruction data of the (N+1)-th region by correcting the scanning data of the (N+1)-th region. The image reconstruction module 620 may further generate reconstruction data of the (N+1)-th region by correcting the initial reconstruction data of the (N+1)-th region based on the first scanning data. More descriptions regarding the generation of reconstruction data may be found elsewhere in the present disclosure, e.g., FIG. 7 and relevant descriptions thereof.

It should be noted that the above description is merely provided for the purposes of illustration, and is not intended to limit the scope of the present disclosure. Apparently, for persons having ordinary skills in the art, multiple variations and modifications may be conducted under the teachings of the present disclosure. However, those variations and modifications do not depart from the scope of the present disclosure. For example, the scanning module 610 and the image reconstruction module 620 may be integrated into a single module. As another example, some other components/modules (e.g., a storage module (not shown) for storing data) may be added into the processing device 120.

FIG. 7 is a schematic flowchart illustrating an exemplary process 700 for scanning a subject according to some embodiments of the present disclosure. In some embodiments,the process 700 may be executed by the scanning system 100. For example, the process 700 may be implemented as a set of instructions (e.g., an application) stored in the storage device 130, and the processing device 120 (e.g.,the modules in FIG. 6) may execute the set of instructions and may accordingly be directed to perform the process 700.

In 710, the processing device 120 (e.g., the scanning module 610) may control a detector to scan an N-th region to obtain scanning data of the N-th region. N may be a positive integer greater than 1. The N-th region may be the aforementioned first region or second region described in FIG. 3 or FIG. 5. For example, as shown in FIG. 5, when N is equal to 1, the N-th region is the first region for canning the head of the subject. As another example, as shown in FIG. 5, when N is equal to 2, the N-th region is the second region for canning the middle body of the subject. In some embodiments, the processing device 120 may scan the N-th region using a similar scanning manner described in the process 300 or FIG. 5, which will not be elaborated on further here. In some embodiments, the scanning module 610 may determine whether the scanning of the subject is completed.

In 720, the processing device 120 (e.g., the image reconstruction module 620) may generate reconstruction data of the N-th region by correcting the scanning data of the N-th region. For example, the processing device 120 may correct the scanning data of the N-th region based on correction information. In some embodiments, the correction information may include electrocardiogram (ECG) information, motion information of the subject (e.g., the head, hand, foot, etc.), or the like, or any combination thereof. Correspondingly, if the correction information is the ECG information, the ECG information may be acquired by an ECG device. If the correction information is the motion information, the motion information may be determined using techniques such as a centroid of distribution (COD)-based algorithm, a proper orthogonal decomposition (POD) algorithm, a principal components analysis (PCA) algorithm, etc. According to some embodiments of the present disclosure, by correcting the scanning data of the N-th region using the correction information, the reconstruction data of the N-th region can be prevented from the bias caused by the movement of the subject's body parts during the scanning of the N-th region.

In 730, the processing device 120 (e.g., the scanning module 610) may cause the detector to move from the N-th region to an (N+1)-th region to obtain first scanning data acquired by the detector during a period when the detector moves from the N-th region to the (N+1)-th region. It should be noted that in the present disclosure, controlling/causing the detector to move from the N-th region to the (N+1)-th region may refer to controlling the detector to transition from scanning the N-th region to scanning the (N+1)-th region. In some embodiments, the processing device 120 may control the detector to transition from scanning the N-th region to scanning the (N+1)-th region using a similar manner described in the process 300 or FIG. 5, which will not be elaborated on further here.

In 740, the processing device 120 (e.g., the scanning module 610) may control the detector to scan the (N+1)-th region to obtain scanning data of the (N+1)-th region. The processing device 120 may scan the (N+1)-th region using a similar scanning manner described in the process 300 or FIG. 5, which will not be elaborated on further here. It should be noted that, if the (N+1)-th region is region where a dimension of the subject along the width direction of the table is greater than the preset scanning width of the detector along the width direction of the table, the processing device 120 may correct scanning data of a next sub-region based on scanning data acquired by the detector during a period when the detector moves from a current sub-region to the next sub-region. In some embodiments of the present disclosure, the N-th region may be a sub-region of the second region, and the (N+1)-th region may be a next sub-region of the second region.

In 750, the processing device 120 (e.g., the image reconstruction module 620) may generate initial reconstruction data of the (N+1)-th region by correcting the scanning data of the (N+1)-th region. A correction manner used to correct the scanning data of the (N+1)-th region to generate the initial reconstruction data of the (N+1)-th region may be similar to the correction manner used to correct the scanning data of the N-th region, which is not repeated here.

In 760, the processing device 120 (e.g., the image reconstruction module 620) may generate reconstruction data of the (N+1)-th region by correcting the initial reconstruction data of the (N+1)-th region based on the first scanning data. According to some embodiments of the present disclosure, by correcting the initial reconstruction data of the (N+1)-th region based on the first scanning data, the reconstruction data of the (N+1)-th region can be prevented from the bias caused by the movement of the subject's body parts during the period when the detector moves from the N-th region to the (N+1)-th region.

In 770, the processing device 120 (e.g., the scanning module 610) may determine whether the scanning of the subject is completed. In response to a determination that the scanning of the subject is completed, the processing device 120 may end the process 700 and perform a next operation, such as reconstructing an image of the subject based on all generated reconstruction data of the subject. In response to a determination that the scanning of the subject does not completed, the processing device 120 may proceed to perform operation 730 to cause the detector to move from the current region to a next region and finally obtain reconstructed data of the next region. The processing device 120 may repeatedly perform operations 730 to 760 until the scanning of the subject is completed.

Having thus described the basic concepts, it may be rather apparent to those skilled in the art after reading this detailed disclosure that the foregoing detailed disclosure is intended to be presented by way of example only and is not limiting. Various alterations, improvements, and modifications may occur and are intended to those skilled in the art, though not expressly stated herein. These alterations, improvements, and modifications are intended to be suggested by this disclosure and are within the spirit and scope of the exemplary embodiments of this disclosure.

**Claims**

1. A method for scanning a subject, implemented on a scanning system having a table capable of moving and a detector capable of moving along at least one of a moving direction of the table or a width direction of the table, the method comprising:

   determining reference dimensions, along the width direction of the table, of the subject on the table at positions along the moving direction of the table;

dividing, based on the reference dimensions, the subject into at least one region along the moving direction of the table, the at least one region including at least one of a first region or a second region, wherein the first region includes a region where a dimension of the subject along the width direction of the table is smaller than or equal to a preset scanning width of the detector along the width direction of the table, and the second region includes a region where a dimension of the subject along the width direction of the table is greater than the preset scanning width of the detector along the width direction of the table; and

controlling a relative motion between the detector and the table to perform scanning on the at least one region in sequence along the moving direction of the table.

2. The method of claim 1, wherein the at least one region includes the first region and the second region.

3. The method of claim 2, wherein the controlling a relative motion between the detector and the table to perform scanning on the at least one region includes:

   controlling a movement of the table to achieve the relative motion when scanning the first region; and
   controlling a movement of the detector to achieve the relative motion when scanning the second region.

4. The method of claim 3, wherein the second region has a first end and a second end along the width direction of the table, and the controlling a movement of the detector to achieve the relative motion when scanning the second region includes:
   controlling the detector to move from the first end to the second end along the width direction of the table.

5. The method of claim 3, wherein the second region includes a first sub-region and a second sub-region adjacent to each other along the moving direction of the table, each of the first sub-region and the second sub-region has a first end and a second end along the width direction of the table, and the first end of the first sub-region and the first end of the second sub-region are located on a same side of the subject, wherein the controlling a movement of the detector to achieve the relative motion when scanning the second region includes:

   controlling the detector to move from the first end of the first sub-region to the second end of the first sub-region to scan the first sub-region;
causing the detector to move from the second end of the first sub-region to the second end of the second sub-region; and
controlling the detector to move from the second end of the second sub-region to the first end of the second sub-region to scan the second sub-region.

6. The method of claim 5, wherein the causing the detector to move from the second end of the first sub-region to the second end of the second sub-region includes:

   controlling the table to move, or
   controlling the detector to move along the moving direction of the table.

7. The method of claim 1, wherein the first region and the second region are arranged in sequence along the moving direction of the table, the controlling a relative motion between the detector and the table to perform scanning on the at least one region includes:

   after the detector completes scanning the first region, controlling the table to move such that a scaning range of the detector transitions from the first region to the second region;
   controlling the detector to move from an initial position of the detector to a first end of the second region; and
   controlling the detector to move from the first end of the second region to a second end of the second region to scan the second region, wherein a distance between the first end and the initial position is less than or equal to a distance between the second end and the initial position.

8. The method of claim 7, wherein the method further comprises:

   after the detector completes scanning the second region, controlling the detector to move back to the initial position of the gantry; and
   controlling the table to move to scan a next first region.

9. The method of claim 1, wherein the determining reference dimensions, along the width direction of the table, of the subject on the table at positions along the moving direction of the table includes:

   obtaining a contour of the subject; and
   determining the reference dimensions based on the contour of the subject.

**10.** The method of claim 9, wherein the obtaining a contour of the subject includes:
obtaining the contour of the subject by using a computed tomography (CT) device or a camera.

**11.** The method of claim 1, wherein the controlling a relative motion between the detector and the table to perform scanning on the at least one region includes:

determining a target motion parameter set based on the at least one region, the target motion parameter set including motion parameters of the detector and the table at each time point; and
controlling the detector and the table to perform the scanning based on the target motion parameter set.

**12.** The method of claim 11, wherein the determining a target motion parameter set based on the at least one region includes:
determining the target motion parameter set based on a constraint condition and an optimization objective, wherein the constraint condition includes a coverage ratio of a total scanning area to an area of the subject being greater than 100%, and the optimization objective includes minimizing a total scanning time.

**13.** The method of claim 11, further comprising:

monitoring a movement of the subject during a scanning process; and
updating the target motion parameter set based on the movement of the subject.

**14.** A single-photon emission computed tomography (SPECT) system, comprising:

a gantry;
a table, configured to carry a subject and move relative to the gantry;
a detector, disposed on the gantry, the detector including at least two grammar detectors; and
a processor, configured to:

acquire a contour of the subject carried on the table,
determine a truncated field of view (FOV) of the detector based on the contour, and
control the at least two grammar detectors to move to positions corresponding to multiple viewing angles relative to the table in the truncated FOV, so as to restore the truncated FOV to a full FOV.

**15.** A single-photon emission computed tomography (SPECT) system, comprising:

a gantry;
a table, configured to carry a subject and move relative to the gantry;
a detector, disposed on the gantry, the detector including a first gamma detector and a second gamma detector arranged opposite to each other;
a processor, configured to:

obtain a contour of the subject lied on the table,
determine a truncated field of view (FOV) of the detector based on the contour, and
control a relative motion between the detector and the table to restore the truncated FOV to a full FOV.

**100**

**FIG. 1A**

Full FOV
region

Truncated region
FOV

Full FOV
region

**FIG. 1B**

**120**

| Dimension determination module | 210 |

| Region division module | 220 |

| Control module | 230 |

**FIG. 2**

**300**

Determining dimensions, along the width direction of the table, of the subject on the table at positions along the moving direction of the table ~ 310

Dividing, based on the dimensions, the subject into at least one region along the moving direction of the table ~ 320

Controlling a relative motion between the probe and the table to perform a scanning on the at least one region in sequence along the moving direction of the table ~ 330

**FIG. 3**

30

40

**FIG. 4A**

Position 2

Position 1

Position 3

DET 1

DET 1

DET 1

Y

X

O

torso

Motion trajectory
of detector

**FIG. 4B**

Motion trajectory
of detector

Position 2

Position 1

DET 1

DET 1

DET 1

DET 1

Position 3

Y

X

O

torso

**FIG. 4C**

**FIG. 4D**

Inner wall of bore

Detector

Z

X

O

First
position

Table

Second
position

Moving direction of table

**FIG. 4E**

**FIG. 5**

<u>**120**</u>

Scanning module ⌇610

Image reconstruction module ⌇620

**FIG. 6**

700

Controlling a detector to scan an N-th region to obtain scanning data of the N-th region — 710

Generating reconstruction data of the N-th region by correcting the scanning data of the N-th region — 720

Causing the detector to move from the N-th region to an (N+1)-th region to obtain first scanning data acquired by the detector during a period when the detector moves from the N-th region to the (N+1)-th region — 730

Controlling a detector to scan the (N+1)-th region to obtain scanning data of the (N+1)-th region — 740

Generating initial reconstruction data of the (N+1)-th region by correcting the scanning data of the N-th region — 750

Generating reconstruction data of the (N+1)-th region by correcting the initial reconstruction data of the (N+1)-th region based on the first scanning data — 760

Does the scanning of the subject be completed? — 770

No

Yes

End

**FIG. 7**

## EUROPEAN SEARCH REPORT

**Application Number**

EP 25 21 9062

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 0 747 007 B1 (HOLOGIC INC [US]) 19 November 2003 (2003-11-19) | 1,11-13 | INV. A61B6/04 |
| A | * paragraphs [0014] - [0039]; claims 1-8; figures 1,2,2a-b,5-7 * | 2-10,14, 15 | A61B6/00 |
| X | US 2024/277306 A1 (ROTH NATHANIEL [IL] ET AL) 22 August 2024 (2024-08-22) | 1-3,9, 10,14,15 | |
| Y | * paragraphs [0399] - [0407]; figures 1,13-15 * | 4-8 | |
| Y | US 2015/094573 A1 (BOUHNIK JEAN-PAUL [IL] ET AL) 2 April 2015 (2015-04-02) * paragraphs [0003] - [0004], [0050] - [0056], [0068], [0072]; figures 1-4,13-14 * | 4-8 | |
| A | US 2017/119322 A1 (YAMADA YASUNOBU [JP] ET AL) 4 May 2017 (2017-05-04) * paragraphs [0081] - [0089]; figures 5,6a-c * | 1-15 | |

**TECHNICAL FIELDS SEARCHED (IPC)**

A61B
G01N

The present search report has been drawn up for all claims

| Place of search | Date of completion of the search | Examiner |
|---|---|---|
| Munich | 23 March 2026 | Daoukou, Eleni |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**EP 4 751 653 A1**

## ANNEX TO THE EUROPEAN SEARCH REPORT
## ON EUROPEAN PATENT APPLICATION NO.

EP 25 21 9062

23-03-2026

| Patent document cited in search report | | Publication date | Patent family member(s) | | Publication date |
|---|---|---|---|---|---|
| EP 0747007 | B1 | 19-11-2003 | DE | 69630733 T2 | 23-09-2004 |
| | | | EP | 0747007 A1 | 11-12-1996 |
| | | | EP | 1366713 A1 | 03-12-2003 |
| | | | US | 5715820 A | 10-02-1998 |
| US 2024277306 | A1 | 22-08-2024 | EP | 2846695 A2 | 18-03-2015 |
| | | | EP | 3647822 A2 | 06-05-2020 |
| | | | US | 2015119704 A1 | 30-04-2015 |
| | | | US | 2018000431 A1 | 04-01-2018 |
| | | | US | 2020015763 A1 | 16-01-2020 |
| | | | US | 2021259644 A1 | 26-08-2021 |
| | | | US | 2021259645 A1 | 26-08-2021 |
| | | | US | 2023128803 A1 | 27-04-2023 |
| | | | US | 2024173003 A1 | 30-05-2024 |
| | | | US | 2024277306 A1 | 22-08-2024 |
| | | | WO | 2013168111 A2 | 14-11-2013 |
| US 2015094573 | A1 | 02-04-2015 | NONE | | |
| US 2017119322 | A1 | 04-05-2017 | NONE | | |

EPO FORM P0459

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 202411718745